# EUROPEAN PATENT SPECIFICATION

(11) **EP 3 110 398 B1**
(45) Date of publication and mention of the grant of the patent: **11.07.2018**
(21) Application number: 15709726.2
(22) Date of filing: 25.02.2015
(51) Int. Cl.: A61K 31/465, A61K 9/00, A61M 15/06, A61M 15/00

(54) **A PROCESS FOR PREPARING A NICOTINE-CONTAINING FORMULATION**
VERFAHREN FÜR DIE ZUBEREITUNG EINER NIKOTINHALTIGEN FORMULIERUNG
PROCÉDÉ DE PRÉPARATION D'UNE FORMULATION CONTENANT DE LA NICOTINE

(30) Priority: 26.02.2014 GB 201403356
(43) Date of publication of application: 04.01.2017
(73) Proprietor: Kind Consumer Limited, London EC1R 5AR (GB)
(72) Inventor: SILCOCK, Alan, London SE1 8TG (GB)
(74) Representative: Boult Wade Tennant
(86) International application number: PCT/GB2015/000069
(87) International publication number: WO 2015/128599

(56) References cited:
- WO-A1-2010/107613
- WO-A1-2011/034723
- WO-A2-2009/001085
- GB-A- 1 528 391

## Description

### Field of the Invention

The invention relates to a process for preparing a formulation comprising nicotine and a liquid propellant gas.

### Background

Some non-combustible products are known in which nicotine is delivered as an aerosol. In some of these products, the nicotine is formulated with a propellant.

### Summary

In accordance with a first aspect of the present invention there is provided a continuous or semi-continuous process for preparing a formulation comprising a mixture of nicotine and a liquefied propellant gas, wherein the components of the formulation are introduced into a mixing vessel in a controlled manner.

In some embodiments, control of the introduction of components of the formulation into the mixing vessel is provided by a means for monitoring the concentration of one or more components of the formulation in the mixing vessel; a means for altering the rate and/or amount of introduction of one or more components of the formulation into the mixing vessel; and a means of providing feedback from the monitoring means to the means for altering the rate and/or amount of introduction of one or more components of the formulation.

In some embodiments, the means for monitoring the concentration of one or more components of the formulation in the mixing vessel and the means for providing feedback from the monitoring process is provided by process analytical technology, and may comprise the use of infrared or ultraviolet spectroscopy.

In some embodiments, the means for altering the rate and/or amount of introduction of one or more components of the formulation is provided by one or more batch or flow meters.

In some embodiments, the liquid propellant gas is an HFA propellant, and may be HFA 134a, HFA152a, HFA 227, HFO1234yf (2,3,3,3-tetrafluoropropene) or HFO1234ez (trans 1,3,3,3-tetrafluoropropene).

In some embodiments, the amount of liquid propellant gas introduced to the mixing vessel is between about 95.0 to 99.5% by weight of the components of the formulation, and/or the amount of nicotine introduced to the mixing vessel is about between 0.05 and 0.1% by weight of the components of the formulation.

In some embodiments, the formulation may further comprise one or more co-solvents. In some embodiments, the co-solvents may be one or both of propylene glycol and ethanol.

In some embodiments, the formulation may further comprise one or more flavourants, one or more humectants and/or one or more sugars and/or sugar substitutes.

In some embodiments, the sugar substitute may be saccharin, and/or the flavourant may be mint, menthol, vanillin or trans-anethole.

In some embodiments, the amount of co-solvent introduced to the mixing vessel may be about 1.5% by weight of the components of the formulation; the amount of flavourant introduced to the mixing vessel may be about 0.1% by weight of the components of the formulation; and the amount of sugar or sugar substitute introduced to the mixing vessel may be about 0.1% by weight of the components of the formulation.

In some embodiments, all of the components of the formulation may be liquid upon introduction to the mixing chamber and/or all of the components of the formulation may be maintained in a liquid form essentially throughout the process.

### Brief Description of the Drawings

Embodiments of the invention will now be described, by way of example only,
with reference to the accompanying drawings, in which:
Figure 1 is a schematic of a process according to the first aspect of the present invention.
Figure 2 is a schematic drawing of a device according to a fourth aspect of the invention, which includes a container containing a formulation prepared by the process according to the first aspect of the invention.

### Detailed Description

Nicotine (C₁₀H₁₄N₂) is a colourless to pale yellow, volatile, oily, hygroscopic liquid, the physiologically active form of which is the S-(-) isomer. It is the chief alkaloid in tobacco products, and acts as a stimulant in mammals. It rapidly crosses the blood-brain barrier, and acts at nicotinic acetylcholine receptors which are present in many tissues in the body, including the central and peripheral nervous system.

Non-combustible devices that deliver nicotine may include systems which comprise a liquid propellant gas, wherein a liquid nicotine-containing formulation is combined with a liquid propellant gas and held, under pressure, in a cartridge or canister. When a valve in the cartridge or canister is opened, the contents are forced out of a small aperture and emerge as an aerosol cloud. The droplets of propellant evaporate rapidly, leaving the nicotine-containing formulation suspended as very fine particles or droplets, which are then inhaled by the user. The size of the particles of droplets can be controlled by varying the size of the aperture.

Hydrofluoroalkane (HFA) propellants, particularly 1,1,1,2-tetrafluoroethane (HFA 134a),1,2-difluoroethane (HFA152a) and 1,1,1,2,3,3,3,-heptafluoropropane (HFA 227), are currently favoured as non-ozone depleting alternatives to the CFC propellants for respiratory delivery of an active agent or drug. Other alternatives to CFCs have been proposed, including dimethyl ether and low molecular weight hydrocarbons, such as propane and butane. Hydrofluoroolefin (HFO) propellants, particularly 2,3,3,3-tetrafluoropropene (HFO1234yf) and trans 1,3,3,3-tetrafluoropropene (HFO1234ez) have also been proposed as propellants with low environmental risk.

Canisters or containers comprising nicotine and a propellant may be designed for direct use, for example the canister or container may comprise an activation mechanism, such as a metering valve, which, when depressed, delivers a measured amount of nicotine, thereby allowing self-administration by the end user. Alternatively, canisters, cartridges or containers comprising nicotine and a propellant can be incorporated into a device, for example by assembling the canister, cartridge or container with a mouthpiece and activation mechanism in order to facilitate self-administration of a dose of nicotine by the end user.

Devices comprising canisters containing nicotine and a liquid propellant gas for inhalation are known in the art. For example, WO 2010/145894 and WO 2011/147691 disclose aerosol generator devices which may be used to deliver a nicotine containing aerosol.

Conventional processes of manufacture for liquid propellant containing canisters are generally characterized as either "pressure filling" or "cold filling".

In cold filling, the propellant, which has been chilled below its boiling point, and remaining components of the formulation, are added to a canister capable of withstanding the vapour pressure of the propellant, and a valve is fitted to the canister. This method has the disadvantage of requiring cooling of the propellant.

In pressure filling, the components of the formulation apart from the propellant are placed in a suitable canister and fitted with a metering valve. The propellant is then forced as a liquid through the valve into the container. WO 2006/004646 provides an example of such a method, wherein a mixture of nicotine and other ingredients are introduced to a canister, which is sealed and then filled with a liquid propellant via a valve, using an automated pressurized-liquid propellant metering system.

In an alternate process of pressure filling, discrete volumes of the components of the formulation, including the propellant, are introduced, in their desired ratios, to a large (typically 550-1000 kg batch capacity) vessel, thoroughly mixed and then, with continuous stirring, dispensed into the canisters or cartridges. This is known as a batch process.

The phrase "a continuous process" as used herein means a method which occurs without interruption, wherein the materials being processed are continuously in motion. A continuous process is typically in operation 24 hours per day, seven days per week with infrequent maintenance shutdowns. Typically a start up or shut down will take several hours. A "semi-continuous" processes can be shut down and re-started more easily, and so can be operated over shorter time periods. A continuous or semi-continuous process requires continuous, controlled input of the components of the formulation.

The terms "controlled input" and "controlled manner" as used herein refer to the use of a means of monitoring and adjusting input (amount and/or rate) of the components in the formulation to the mixing vessel, wherein the means provides feedback which, when appropriate, automatically results in the alteration of one or more of the input parameters in order to effect a change in the amount and/or rate of one or more of the components entering the mixing vessel.

Suitable monitoring and feedback means can be provided by the use of process analytical technology (PAT). PAT typically comprises defining the Critical Process Parameters (CPPs) of the equipment used to make the product, which in turn affect the Critical Quality Attributes (CQAs), and then controlling the CPPs within defined limits. CPPs are the independent process parameters that may affect the quality of the product (or intermediate) resulting from the process. CQAs are attributes of the process that are critical to the quality of the resultant product (or have a direct and significant impact on its actual or perceived quality). The use of PAT can help to provide a steady state process, as it provides feedback on upstream & downstream events resulting from inherent variables in the components of the formulation (for example moisture content), or processing equipment (inherent tolerance) and/or changes in the input parameters. As a result, common causes of variability are easier to identify, monitor and compensate for.

PAT typically requires the use of process analytical chemistry (PAC) tools, such as in-line and/or on-line analytical instruments, to measure the parameters that have been defined as CPP. PAC tools can include infra red spectroscopy (including near infrared spectroscopy), ultraviolet spectroscopy or Raman spectroscopy.

In addition, a PAT system typically comprises data acquisition and analysis tools, such as a multivariate data acquisition and analysis software package, to collect and statistically analyze raw data arising from the process in order to determine the CPP parameters. A PAT system may also comprise continuous improvement tools such as software packages which accumulate quality control data over time, with the aim of defining process weaknesses, and implementing process improvements.

Accordingly, in some embodiments, infrared or ultraviolet spectroscopy is used to monitor, either periodically or continuously, the constitution and/or consistency of the formulation in the mixing vessel, and in particular, the concentration of one or more components of the formulation in the mixing vessel. The results of this monitoring may be used to alter the amount and/or rate of introduction of one or more of the components of the formulation to the mixing vessel, in order to ensure that the constitution and/or consistency of the formulation within the mixing chamber stays within the specifications. For example, data provided as a result of infrared analysis may be fed back to one or more batch or flow meters, which, as a result, alter the rate and/or amount of one or more components being introduced into the mixing vessel.

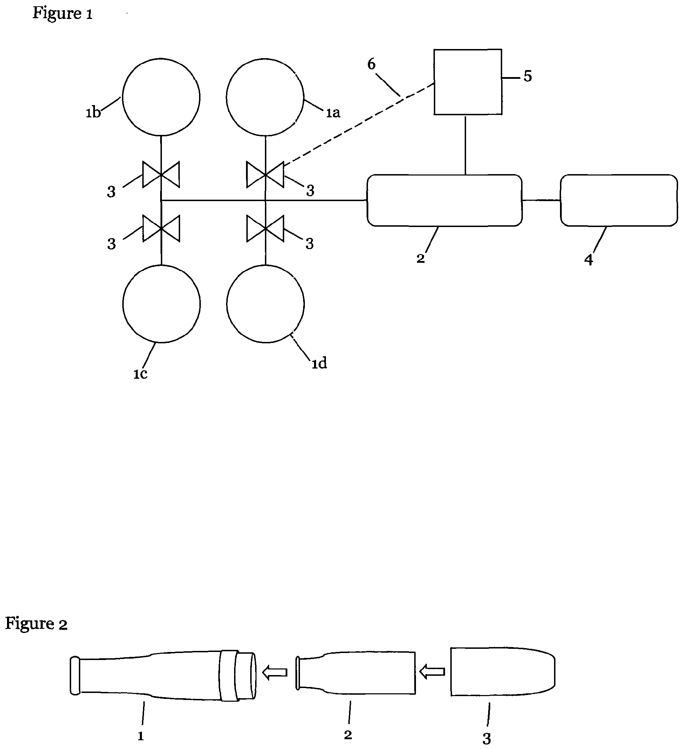

Referring to Figure 1, four containers 1a-1d are illustrated, each of which holds one or more components of a formulation according to the present invention.

Container 1a holds nicotine. The term "nicotine" as used herein, includes all geometric or stereoisomeric forms of nicotine, including cis- and trans isomers, diastereomers, R and S enantiomers, mixtures and racemic mixtures thereof, as well as derivative, metabolites or analogues of nicotine that exhibits properties similar to nicotine, such as norcotinine, cotinine and nornicotine. The term "nicotine" as used herein also includes a nicotine-containing solution, for example, a nicotine-containing tobacco extract. Nicotine for use in the present invention may be isolated from natural sources or synthetically produced. In some embodiments, the nicotine may be present in salt form.

In some embodiments, the nicotine may be in liquid form prior to or upon introduction to the mixing vessel.

Container 1b in Figure 1 holds the propellant. The term "propellant" as used herein means one or more liquids or gases which exert a vapour pressure at room temperature sufficient to propel the formulation from the cartridge or canister to the user on actuation. The propellant may be a pharmacologically inert liquid or gas.

In some embodiments, suitable propellants include, for example, hydrofluoroalkanes such as 1,1,1,2-tetrafluoroethane (CF₃CH₂F) (HFA-134a) and 1,1,1,2,3,3,3-heptafluoropropane (CF₃CHFCF₃) (HFA-227), perfluoroethane, monochlorodifluoromethane, 1,1-difluoroethane (HFA-152a), tetrafluoromethane (PFC-14), trifluoromethane (HFA-23), difluoromethane (HFA-32), fluoromethane (HFA-41), 1,1,2,2,2-pentafluoroethane (HFA-125), 1,1,2,2-tetrafluoroethane (HFA-134), decafluorobutane (CF₃CF₂CF₂CF₃); hydrofluoroolefins such as 2,3,3,3-tetrafluoropropene (HFO1234yf) and trans 1,3,3,3-tetrafluoropropene (HFO1234ez); dialkyl ethers such as dimethyl ether; and low molecular weight hydrocarbons such as n-butane, iso-butane, and propane.

In some embodiments, the propellant may be HFA-227 or HFA-134a or a mixture thereof. In some embodiments, the propellant is HFA-134a.

In some embodiments, the propellant may be in a substantially or entirely liquid state as it is mixed with the other components of the formulation. In some embodiments, the propellant may be in a substantially or entirely liquid state essentially throughout the process. In order to achieve this, the propellant may be maintained under pressure during each stage of the process. Accordingly, the propellant may be maintained at between 6-10 bar, and around 20°C during each stage of the process.

In some embodiments, the formulation may further comprise one or more natural or artificial flavourants.

As used herein, the terms "flavour" and "flavourant" refer to materials which, where local regulations permit, may be used to create a desired taste or aroma in a product for adult consumers.

In some embodiments, the flavour or flavourant may be menthol, citrus, vanillin, aniseed, transanethole, benzaldehyde or acetylaldehyde.

In some embodiments, the formulation may further comprise one or more sugars or sugar substitutes. For example, in some embodiments, the formulation may comprise dihydrochalcones, monellin, steviosides, dihydroflavenol, maltitol, xylitol; water-soluble artificial sweeteners such as soluble saccharin salts, for example, sodium or calcium saccharin salts, acesulfame-K; dipeptide based sweeteners, such as L-aspartic acid derived sweeteners or aspartame; water-soluble sweeteners including naturally occurring water-soluble sweeteners such as chlorinated derivates of sucrose, for example chlorodeoxysucrose or chlorodeoxygalactosucrose; protein based sweeteners such as talin or thaumatin I or II; and monatin and its derivatives. In some embodiments, the sugar substitute is saccharin.

In some embodiments, the components of the formulation may be in substantially or entirely liquid form prior to or upon introduction to the mixing vessel. Accordingly, any ingredients in solid form may be dissolved prior to addition to the mixing vessel. To achieve this aim, the formulation may comprise a co-solvent.

The term "co-solvent" as used herein refers to a substance in which one or more of the other components of the formulation dissolve. Co-solvents are typically less volatile than the propellant used and may dissolve a compound in the propellant, lower the vapour pressure of the propellant system and/or promote miscibility between propellants and immiscible solvents.

In some embodiments, the co-solvent is one or more of propylene glycol, alcohols, such as ethanol or isopropyl alcohol, glycerol, polyethylene glycol or dextran. In some embodiments, the co-solvents are propylene glycol and ethanol.

Container ic in Figure 1 holds propylene glycol.

Menthol and saccharin, which are typically in solid form at room temperature, may be dissolved in ethanol and/or propylene glycol prior to introduction to the mixing vessel 2.

Container 1d in Figure 1 holds ethanol, saccharin and menthol.

Alternatively, a pre-mixture of menthol, saccharin and ethanol may be mixed with nicotine and propylene glycol, and held in a single container prior to introduction to the mixing vessel

In some embodiments, the formulation may comprise one or more humectants. The humectant may also act as a co-solvent. In some embodiments the humectant is propylene glycol.

As discussed above, the amount and/or rate of introduction of the components to the mixing vessel is controlled via the use of one or more batch and/or flow meters. Accordingly, in Figure 1, each of the components, or pre-mixtures of the components in the formulation are introduced to the mixing vessel 2 via a batch meter 3. The concentration of one or more components in the mixing vessel 2 may be monitored using a monitoring system 5 which provides feedback (via 6 in Figure 1) to at least one, and typically all batch or flow meters 3 in the system.

In some embodiments, the formulation may, by weight of the components of the formulation, comprise less than 1% nicotine, less than 0.5% nicotine, less than 0.2% nicotine, and less than 0.1% nicotine. In some embodiments the formulation may comprise about 0.056% nicotine by weight of the components of the formulation.

In some embodiments, the formulation may, by weight of the components of the formulation, comprise 90% propellant, between 90-95% propellant, between 95 and 99.5%, 95-98% or 98-99% propellant. In some embodiments, the formulation may comprise about 98.5% propellant by weight of the components of the formulation.

In some embodiments, the formulation may, by weight of the components of the formulation, comprise less than 1% flavourant, less than 0.5% flavourant, less than 0.2% flavourant, or less than 0.1% flavourant.

In some embodiments, the formulation may, by weight of the components of the formulation, comprise less than 1%, less than 0.5%, less than 0.2%, or less than 0.1% sugar or sugar substitute.

In some embodiments, the formulation may by weight of the components of the formulation, comprise between 1-5% co-solvent, between 1-3% co-solvent or about 1.5% co-solvent.

In some embodiments the formulation may, by weight of the components of the formulation, comprise less than 2% propylene glycol, less than 1% propylene glycol or less than about 0.5% propylene glycol.

In some embodiments, the formulation may, by weight of the components of the formulation, comprise between 1-5% humectant, between 1-3% humectant or about 1.5% humectant.

In some embodiments, the formulation may, by weight of the components of the formulation, comprise less than 2% ethanol, less than 1.5% ethanol or about 1% ethanol.

The components of the formulation are then mixed within the mixing vessel 2. In some embodiments, the mixing process is carried out under pressure, typically under around 6-10 bar pressure. In addition, the temperature of the process may be maintained at around 20°C.

Typically, a high degree of mixing is required, and this may advantageously be achieved using a dynamic mixing system. In some embodiments, an in-line mixing device may be used. In some embodiments, a throughput for the mixing stage of approximately 1, 2, 5, or 7.5 kg per minute may be achieved.

The mixed formulation then exits the mixing vessel and enters a filling line 4 from where it is dispensed into canisters. In some embodiments, the formulation may continue to be mixed or stirred following exit from the mixing vessel. In some embodiments, the filling line may be a high capacity filling line such as a minicentomat™ filling line. In certain embodiments, the throughput of filled canisters achieved is more than 100 per minute, more than 150 per minute, more than 200, 210, 220 or 230 per minute, or at least 240 canisters per minute.

In some embodiments, all of the components of the formulation may be in substantially or entirely liquid form throughout the process according to the present invention. In order to achieve this, the system may be maintained under pressure during each stage of the process. Accordingly, the filling line may be maintained at around 6 bar pressure and a temperature of about 20°C.

In some embodiments, the pressure may be regulated to 10 bar pressure. In some embodiments, all of the equipment used in the process may be regulated to 10 bar pressure.

As the skilled person would recognize, suitable commercially available mixing and dispensing systems may be used to carry out a process according to the first aspect of the invention. For example, equipment typically provided for hydrogenation reaction chemistry, such as that provided by AM Technology, could be used to provide a continuous process in accordance with the first aspect of the present invention.

The provision of a continuous or semi-continuous process to produce a formulation comprising nicotine and a propellant has several advantages. For example, a batch processes involves idle time or downtime between batches whilst the equipment is reconfigured, and its output tested before the next batch can be produced. In addition, it can be difficult to empty the vessel completely using a batch process. For example, a 550 kg vessel gives a theoretical yield of 26,900 canisters before the batch is exhausted. However, a "heel" of around 37 kg remains in the vessel, which reduces the yield from a batch to around 25,000 canisters. In comparison, a process according to the present invention is efficient and economical, as lost operating time is minimized.

Furthermore, use of a batch process with a 550 kg mixing vessel to produce canisters comprising a formulation comprising nicotine and a propellant gives rise to around 30 million canister per annum (working on a 6 day, three shift pattern). In comparison, a process according to the present invention, when run on the same 6 day three shift pattern, gives rise to around 80-90 million canisters per annum. In addition, use of a batch process results in an increase in the head space in the vessel as the canisters are filled, with the result that the aerosol, which is highly volatile, evaporates. As a result, the nicotine content increases in the remaining mixture. At least some of the embodiments described above can facilitate mitigation or overcoming of this issue.

A process according to the present invention has not, to date, been envisaged. Whilst the equipment used to carry out a process according to the invention is commercially available, such equipment is typically used for chemical reactions, such as hydrogenation, oxidation, Grignard, and Hoffmann reactions, not to effect mixing, and certainly not to effect mixing of a formulation comprising a propellant.

A canister or container may be filled with a formulation prepared by a process as described. Suitable containers may be made of any material capable of withstanding the vapour pressure of the propellant used. In certain embodiments, the container may be made of plastic or aluminium. In certain embodiments, the canister may comprise a valve, an activation mechanism and a mouth- and/or nosepiece, to allow administration of the formulation contained therein to the user.

A canister or container comprising a formulation may be included in a device. The device may further comprise an activation mechanism and a mouth and/or nose piece, to allow administration of the formulation contained therein to the user.

The device illustrated in Figure 2 comprises a mouth section 1, a container 2, which contains a formulation according to the first aspect of the present invention, and an activation mechanism and cap 3. The device may be assembled, as indicated in Figure 2. The activation mechanism 3 may be depressed, causing it to telescope onto the mouthpiece section of the device 1, and simultaneously compress the container, causing the release of a dose of nicotine, which is propelled from the container 2 to the user via the mouthpiece 1.

The mouth section and cap of the device may be made of any suitable material, for example, plastic.

In certain embodiments, the device may be approximately the size of a smoking article, for example, a cigarette.

The following example is provided to illustrate the present invention and should not be construed as limiting thereof.

### Example

The components of the formulation are introduced into a mixing chamber from four containers (1a-1d), with the batch meters controlling the rate of each component, or mixture of components, to give the desired percentage, as detailed in Table 1, in the ultimate mixture.

**Table 1**

| Container | Formulation component | Approximate amount (%) |
|---|---|---|
| 1a | HFA 134a | 98.54 |
| 1b | Nicotine | 0.06 |
| 1c | Propylene glycol | 0.39 |
| 1d | Ethanol | 0.95 |
| | Menthol | 0.05 |
| | Saccharin | 0.01 |

Alternatively, the components of the formulation are introduced into a mixing chamber from two containers, wherein one container holds a mixture of menthol, saccharin, ethanol, nicotine, and propylene glycol; and the other container holds HFA 134a. The contents of each container are fed into the mixing vessel, via a batch meter, at a ratio of approximately 98.5% HFA: 1.5% of the pre-mixed other components.

In each case, the propellant is held under pressure in liquid form in its container.

The mixture is held in the mixing vessel under 6-10 bar pressure and a temperature of 20°C and mixed thoroughly, before exiting the mixing chamber, under pressure, achieving a throughput of approximately 5 kg per minute, to a high throughput filing line, from where it is dispensed into canisters.

In order to address various issues and advance the art, the entirety of this disclosure shows by way of illustration various embodiments in which the claimed invention may be practiced and provide for a superior process for preparing a formulation comprising nicotine and a liquid propellant gas. The advantages and features of the disclosure are of a representative sample of embodiments only, and are not exhaustive and/or exclusive. They are presented only to assist in understanding and teach the claimed features.

## Claims

1. A continuous or semi-continuous process for preparing a formulation comprising a mixture of nicotine and a liquefied propellant gas, wherein the components of the formulation are introduced into a mixing vessel in a controlled manner.

2. A process according to claim 1, wherein control of the introduction of components of the formulation into the mixing vessel is provided by a means for monitoring the concentration of one or more components of the formulation in the mixing vessel; a means for altering the rate and/or amount of introduction of one or more components of the formulation into the mixing vessel; and a means of providing feedback from the monitoring means to the means for altering the rate and/or amount of introduction of one or more components of the formulation into the mixing vessel.

3. A process according to claim 2, wherein the means for monitoring the concentration of one or more components of the formulation in the mixing vessel and the means for providing feedback from the monitoring process is provided by process analytical technology, optionally wherein the process analytical technology comprises the use of infrared or ultraviolet spectroscopy.

4. A process according to claim 2 or 3, wherein the means for altering the rate and/or amount of introduction of one or more components of the formulation is provided by one or more batch or flow meters.

5. A process according to any of the preceding claims, wherein the liquid propellant gas is HFA 134a, HFA152a, HFA 227, HFO1234yf (2,3,3,3-tetrafluoropropene) or HFO1234ez (trans 1,3,3,3-tetrafluoropropene).

6. A process according to any of the preceding claims, wherein the amount of liquid propellant gas introduced to the mixing vessel is between 95.0 and 99.5% by weight of the components of the formulation, and/or the amount of nicotine introduced to the mixing vessel is between 0.05 and 0.1% by weight of the components of the formulation.

7. A process according to any of the preceding claims, wherein the formulation further comprises one or more co-solvents.

8. A process according to claim 7, wherein the co-solvents are one or both of propylene glycol and ethanol.

9. A process according to any of the preceding claims, wherein the formulation further comprises one or more flavourants, one or more humectants and/or one or more sugars or sugar substitutes.

10. A process according to claim 9, wherein the sugar substitutes is saccharin, and/or the flavourant is mint, menthol, vanillin or transanethole.

11. A process according to claim 9 or 10, wherein the amount of co-solvent introduced to the mixing vessel is 1.5% by weight of the components of the formulation; the amount of flavourant introduced to the mixing vessel is 0.1%; and the amount of sugar or sugar substitute introduced to the mixing vessel is 0.1%.

12. A process according to any of the preceding claims, wherein all of the components of the formulation are liquid upon introduction to the mixing chamber, and/or wherein all of the components of the formulation are maintained in liquid form essentially throughout the process.

## Patentansprüche

1. Kontinuierliches oder semi-kontinuierliches Verfahren zur Herstellung einer Formulierung umfassend eine Mischung von Nikotin und einem verflüssigten Treibgas, worin die Komponenten der Formulierung in ein Mischgefäß in kontrollierter Weise eingeführt werden.

2. Verfahren gemäß Anspruch 1, worin für Kontrolle der Einführung der Komponenten der Formulierung in das Mischgefäß durch ein Mittel zur Überwachung der Konzentration einer oder mehrerer Komponenten der Formulierung in dem Mischgefäß, ein Mittel zur Änderung der Rate und/oder der Menge der Einführung einer oder mehrerer Komponenten der Formulierung in das Mischgefäß, und ein Mittel zur Erzeugung einer Rückmeldung von dem Überwachungsmittel an das Mittel zur Änderung der Rate und/oder der Menge der Einführung einer oder mehrerer Komponenten der Formulierung in das Mischgefäß gesorgt wird.

3. Verfahren gemäß Anspruch 2, worin das Mittel zur Überwachung der Konzentration einer oder mehrerer Komponenten der Formulierung in dem Mischgefäß und das Mittel zur Erzeugung einer Rückmeldung aus dem Überwachungsprozess durch Prozessanalysetechnik bereitgestellt wird, optional wobei die Prozessanalysetechnik die Verwendung von Infrarot- oder Ultraviolettspektroskopie umfasst

4. Verfahren gemäß Anspruch 2 oder 3, worin das Mittel zur Änderung der Rate und/oder Menge der Einführung einer oder mehrerer Komponenten der Formulierung durch ein oder mehrere Chargen- oder Durchflussmessgeräte bereitgestellt wird.

5. Verfahren gemäß einem der vorhergehenden Ansprüche, worin das flüssige Treibgas HFA134a, HFA152a, HFA227, HFO1234yf (2,3,3,3-Tetrafluorpropen) oder HFO1234ez (Trans1,3,3,3-tetrafluorpropen) ist.

6. Verfahren gemäß einem der vorhergehenden Ansprüche, worin die Menge des in das Mischgefäß eingeführten flüssigen Treibgases bei zwischen 95,0 und 99,5 Gew.% der Komponenten der Formulierung liegt und/oder die Menge von in das Mischgefäß eingeführtem Nikotin bei zwischen 0,05 und 0,1 Gew.% der Komponenten der Formulierung liegt.

7. Verfahren gemäß einem der vorhergehenden Ansprüche, worin die Formulierung weiter ein oder mehrere Zusatzlösungsmittel umfasst.

8. Verfahren gemäß Anspruch 7, worin die Zusatzlösungsmittel ein oder mehrere von Propylenglykol und Ethanol sind.

9. Verfahren gemäß einem der vorhergehenden Ansprüche, worin die Formulierung weiterhin ein oder mehrere Geschmacksstoffe, ein oder mehrere Befeuchtungsmittel und/oder ein oder mehrere Zucker- oder Zuckerersatzstoffe umfasst.

10. Verfahren gemäß Anspruch 9, worin die Zuckerersatzstoffe Saccharin und/oder der Geschmacksstoff Minze, Menthol, Vanillin oder Transanethol sind.

11. Verfahren gemäß Anspruch 9 oder 10, worin die Menge des in das Mischgefäß eingeführten Zusatzlösungsmittels 1,5 Gew.% der Komponenten der Formulierung, die Menge des in das Mischgefäß eingeführten Geschmacksstoffes 0,1 %, und die Menge von in das Mischgefäß eingeführtem Zucker oder Zuckerersatzstoff 0,1 % beträgt.

12. Verfahren gemäß einem der vorhergehenden Ansprüche, worin alle Komponenten der Formulierung bei der Einführung in die Mischkammer flüssig sind und/oder worin alle Komponenten der Formulierung im Wesentlichen während des gesamten Prozesses in flüssiger Form gehalten werden.

## Revendications

1. Procédé continu ou semi-continu pour la préparation d'une formulation comprenant un mélange de nicotine et d'un gaz propulseur liquéfié, dans lequel les composants de la formulation sont introduits dans une cuve de mélange de manière régulée.

2. Procédé selon la revendication 1, dans lequel la régulation de l'introduction des composants de la formulation dans la cuve de mélange est fournie par un moyen pour surveiller la concentration d'un ou de plusieurs composants de la formulation dans la cuve de mélange ; un moyen pour modifier la vitesse et/ou la quantité d'introduction d'un ou de plusieurs composants de la formulation dans la cuve de mélange ; et un moyen pour fournir un retour d'information à partir du moyen de surveillance jusqu'au moyen pour modifier la vitesse et/ou la quantité d'introduction d'un ou de plusieurs composants de la formulation dans la cuve de mélange.

3. Procédé selon la revendication 2, dans lequel le moyen pour surveiller la concentration d'un ou de plusieurs composants de la formulation dans la cuve de mélange et le moyen pour fournir un retour d'information à partir du procédé de surveillance est fourni par une technologie analytique de procédé, facultativement dans lequel la technologie analytique de procédé comprend l'utilisation d'une spectroscopie infrarouge ou ultraviolette.

4. Procédé selon la revendication 2 ou 3, dans lequel le moyen pour modifier la vitesse et/ou la quantité d'introduction d'un ou de plusieurs composants de la formulation est fourni par un ou plusieurs débitmètres ou compteurs de lot.

5. Procédé selon l'une quelconque des revendications précédentes, dans lequel le gaz propulseur liquide est l'HFA₁₃₄ₐ, l'HFA₁₅₂ₐ, l'HFA₂₂₇, l'HFO_{1234f} (2,3,3,3-tétrafluoropropène) ou l'HFO_{1234ez} (trans 1,3,3,3-tétrafluoropropène) .

6. Procédé selon l'une quelconque des revendications précédentes, dans lequel la quantité de gaz propulseur liquide introduite dans la cuve de mélange est comprise entre 95,0 et 99,5 % en poids des composants de la formulation, et/ou la quantité de nicotine introduite dans la cuve de mélange est comprise entre 0,05 et 0,1 % en poids des composants de la formulation.

7. Procédé selon l'une quelconque des revendications précédentes, dans lequel la formulation comprend en outre un ou plusieurs co-solvants.

8. Procédé selon la revendication 7, dans lequel les co-solvants sont un ou les deux parmi le propylène glycol et l'éthanol.

9. Procédé selon l'une quelconque des revendications précédentes, dans lequel la formulation comprend en outre un ou plusieurs agents aromatisants, un ou plusieurs humectants et/ou un ou plusieurs sucres ou substituts du sucre.

10. Procédé selon la revendication 9, dans lequel le substitut du sucre est la saccharine et/ou l'agent aromatisant est la menthe, le menthol, la vanilline ou le trans-anéthole.

11. Procédé selon la revendication 9 ou 10, dans lequel la quantité de co-solvant introduite dans la cuve de mélange est 1,5 % en poids des composants de la formulation ; la quantité d'agent aromatisant introduite dans la cuve de mélange est 0,1 % ; et la quantité de sucre ou de substitut du sucre introduite dans la cuve de mélange est 0,1 %.

12. Procédé selon l'une quelconque des revendications précédentes, dans lequel tous les composants de la formulation sont liquides lors de l'introduction dans la chambre de mélange, et/ou dans lequel tous les composants de la formulation sont maintenus sous une forme liquide pendant sensiblement tout le procédé.
